# EUROPÄISCHE PATENTANMELDUNG

(11) **EP 4 778 621 A1**
(43) Veröffentlichungstag der Anmeldung: **22.07.2026**
(21) Anmeldenummer: 25152024.3
(22) Anmeldetag: 15.01.2025
(51) Int. Cl.: B01F 35/71, B01F 33/501, B01F 35/75, A61B 17/88, B01F 101/20

(54) **KNOCHENZEMENT-MISCHSYSTEM MIT ZWISCHENKAMMERVENTIL**

(71) Anmelder: Heraeus Medical GmbH, 61273 Wehrheim (DE)
(72) Erfinder: Dr. Kluge, Thomas, 61273 Wehrheim (DE); Dr. Vogt, Sebastian, 61273 Wehrheim (DE)
(74) Vertreter: Heraeus IP

(57) **Zusammenfassung**

Die Erfindung betrifft eine Vorrichtung zur Herstellung eines Knochenzements, aufweisend einen Vorratsbehälter (100) zur Aufnahme eines versiegelten Gefäßes (101) mit einer Monomerflüssigkeit (102), und eine Kartusche (200) zum Mischen von Knochenzement, wobei die Kartusche (200) einen ersten Raum (201) zur Aufnahme von Monomerflüssigkeit aus dem Vorratsbehälter (100) und einen zweiten Raum (202) zur Aufnahme eines Knochenzementpulvers (203) aufweist, und wobei die Kartusche weiterhin ein Ventil (204) aufweist, welches den ersten Raum (201) mit dem zweiten Raum (202) fluidleitend verbindet,
wobei die Vorrichtung ausgebildet und eingerichtet ist,
in einem ersten Schritt innerhalb des Vorratsbehälters (100) eine Monomerflüssigkeit aus einem versiegelten Gefäß (101) freizusetzen und an den ersten Raum (201) der Kartusche abzugeben,
in einem zweiten Schritt die Monomerflüssigkeit innerhalb des ersten Raums (201) zu halten, wobei ein Kontakt der Monomerflüssigkeit mit dem zweiten Raum (202) der Kartusche (200) vermieden wird,
und in einem dritten Schritt die Monomerflüssigkeit aus dem ersten Raum (201) der Kartusche (200) in den zweiten Raum der Kartusche (200) abzugeben.

## Beschreibung

### Gebiet der Erfindung

Die vorliegende Erfindung betrifft das Gebiet der Medizintechnik, insbesondere Vorrichtungen zur Herstellung eines PMMA-Knochenzements und zur Lagerung von Komponenten davon.

### Stand der Technik

Polymethylmethacrylat-Knochenzemente gehen auf die grundlegenden Arbeiten von CHARNLEY zurück. Polymethylmethacrylat-Knochenzemente bestehen üblicherweise aus einer flüssigen Monomerkomponente und einer Pulverkomponente. Die Monomerkomponente kann beispielsweise das Monomer Methylmethacrylat und einen darin gelösten Aktivator (z.B. N,N-Dimethyl-p-toluidin) enthalten. Die Pulverkomponente, auch als Knochenzementpulver bezeichnet, kann ein oder mehrere Polymere aufweisen, die auf Basis von Methylmethacrylat und Comonomeren, wie Styren, Methylacrylat oder ähnlichen Monomeren durch Polymerisation, vorzugsweise Suspensionspolymerisation, herstellbar sein können. Die Pulverkomponente kann weiterhin einen Röntgenopaker und einen Initiator wie z.B. Dibenzoylperoxid aufweisen. Beim Vermischen der Pulverkomponente mit der Monomerkomponente kann durch Quellung der Polymere der Pulverkomponente im Methylmethacrylat ein plastisch verformbarer Teig entstehen, der eigentliche Knochenzementteig. Beim Vermischen der Pulverkomponente mit der Monomerkomponente reagiert beispielsweise der Aktivator N,N-Dimethyl-p-toluidin mit Dibenzoylperoxid unter Bildung von Radikalen. Die gebildeten Radikale können die radikalische Polymerisation des Methylmethacrylates initiieren. Mit fortschreitender Polymerisation des Methylmethacrylates erhöht sich die Viskosität des Zementteigs, bis dieser erstarrt. Polymethylmethacrylat-Knochenzemente können in geeigneten Mischbechern mit Hilfe von Spateln durch Vermischen der Pulverkomponente mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US6,033,105A, US5,624,184A, US4,671,263A, US4,973,168A, US5,100,241A, WO99/67015A1, EP1020167A2, US5,586,821A, EP1016452A2, DE3640279A1, WO94/26403A1, EP1005901A2, US5,344,232A.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen "Full-Prepacked-Mischsysteme" sind in den Patentdokumenten EP0380867B1, EP0796653B1, EP0692229B1, US5997544A, US6709149B1, DE69812726T2 und US5588745A, WO9416951A1, DE19718648A1, EP1741413B1, EP3054880B1, DE102009031178B3, US8662736B2, EP2269718B1, EP2281532B1 und EP3093067B1 beschrieben.

In den Patenten EP3320869B1 und EP3320870B1 wurde dagegen ein Zementiersystem beschrieben, bei denen die Vermischung der Monomerflüssigkeit mit dem Zementpulver durch Einpressen von Monomerflüssigkeit in verdichtetes Zementpulver erfolgt.

### Aufgaben der Erfindung

Ein Vorteil der Mischsysteme gemäß EP3320869B1 und EP3320870B1 besteht darin, dass keine externe Vakuumquelle und keine Mischorgane, wie manuelle bedienbare Rührer, erforderlich sind. Es zeigte sich in eigenen praktischen Versuchen, dass mit einer solchen Vorrichtung zuverlässig Polymethylmethacrylat-Knochenzementteig hergestellt werden kann. Bei diesen Vorrichtungen wird ein Monomerflüssigkeitsbehälter, der in einer Kartusche angeordnet ist, durch Vorschub eines Kolbens zerstört und die Monomerflüssigkeit durch Druckeinwirkung in verdichtetes Polymethylmethacrylat-Knochenzementpulver eingepresst. Das Öffnen des Monomerflüssigkeitsbehälters und das Transferieren der Monomerflüssigkeit in das Polymethylmethacrylat-Knochenzementpulver kann unmittelbar nacheinander erfolgen. Der Hohlraum in der Kartusche, in dem der Monomerflüssigkeitsbehälter angeordnet ist, und in dem sich die aus dem zerborstenen Monomerflüssigkeitsbehälter freigesetzte Monomerflüssigkeit befindet, ist flüssigkeits- und gasleitend durch eine Durchführung mit dem Hohlraum in der Kartusche verbunden, in dem das Polymethylmethacrylat-Knochenzementpulver angeordnet ist. Das bedeutet, das nach Öffnen des Monomerflüssigkeitsbehälters die Monomerflüssigkeit umgehend in den Hohlraum zum Polymethylmethacrylat-Knochenzementpulver transferiert werden sollte, um eine Verklebung des Polymethylmethacrylat-Knochenzementpulvers durch Monomerdämpfe beziehungsweise durch Monomerflüssigkeit zu verhindern, die ungehindert durch die Durchführung zum Polymethylmethacrylat-Knochenzement gelangen können. Monomerflüssigkeit und auch Dämpfe des Monomers quellen die Polymethylmethacrylat-Partikel an und diese gequollenen Partikel verkleben innerhalb kurzer Zeit miteinander, wodurch ein späterer Transfer der Monomerflüssigkeit erschwert oder verhindert wird.

In solchen herkömmlichen Systemen, wie beispielsweise den Mischsystemen gemäß EP3320869B1 und EP3320870B1, ist weiterhin häufig ein hoher Kraftaufwand erforderlich, um einen darin enthaltenen Monomerflüssigkeitsbehälter zu öffnen, und anschließend die daraus freigegebene Monomerflüssigkeit unter Kompression des geöffneten Behälters in ein anderes Kompartiment der Vorrichtung, welches ein Knochenzementpulver enthält, zu überführen. Der von einem Benutzer aufzubringende Kraftaufwand setzt sich somit aus drei Bestandteilen zusammen, nämlich erstens der benötigten Kraft zum Aufbrechen des Monomerflüssigkeitsbehälters, zweitens der benötigten Kraft zur Verdichtung der dabei entstehenden Bruchstücke oder Reste des Behälters, und drittens der benötigten Kraft zur Druckbeaufschlagung der freigegebenen Monomerflüssigkeit, um diese in ein Knochenzementpulver einzupressen.

Eine Aufgabe der Erfindung besteht daher in der Bereitstellung einer verbesserten Vorrichtung zum Lagern und Vermischen von Polymethylmethacrylat-Knochenzement gegenüber den Vorrichtungen gemäß EP3320869B1 und EP3320870B1. Ein wünschenswerte Ausführungsform betrifft ein "Full-Prepack-Mischsystem", bei dem Polymethylmethacrylat-Knochenzementpulver und Monomerflüssigkeit in separaten Kompartimenten gelagert werden und die nach Aktivierung der Vorrichtung in dieser vermischt werden können, ohne dass der medizinische Anwender in Kontakt mit dem Polymethylmethacrylat-Knochenzementpulver und der Monomerflüssigkeit kommt. Die Vorrichtung kann es gestatten, die Monomerflüssigkeit nach Öffnen des Monomerflüssigkeitsbehälters und dem Transfer der freigesetzten Monomerflüssigkeit in einem Kompartiment für einen Zeitraum von wenigen Minuten bis zu einer Stunde zwischenzulagern, ohne dass das Polymethylmethacrylat-Knochenzementpulver mit der Monomerflüssigkeit unbeabsichtigt in Kontakt kommen kann. Der unbeabsichtigte Kontakt der Monomerflüssigkeit mit dem Polymethylmethacrylat-Knochenzementpulver soll bevorzugt verhindert werden. Der Transfer der Monomerflüssigkeit und das Inkontaktbringen der Monomerflüssigkeit mit Knochenzementpulver sollen bevorzugt nur durch eine gezielte Einwirkung des Anwenders ausführbar sein.

Weiterhin ist es wünschenswert, dass beim Austragen des Knochenzements ein möglichst geringer Kraftaufwand benötigt wird. Die vorliegende Erfindung kann dies durch eine Aufteilung des oben beschriebenen Kraftaufwands erreichen.

### BEVORZUGTE AUSFÜHRUNGSFORMEN

Eine Aufgabe der vorliegenden Erfindung besteht darin, eines oder mehrere der oben geschilderten und weitere Probleme des Stands der Technik zu lösen. Beispielsweise liefert die Erfindung eine Vorrichtung zum Lagern und Mischen von Knochenzement, die eine einfache und zuverlässige Mischung einer Monomerflüssigkeit mit einer Pulverkomponente eines PMMA-Knochenzements erlaubt, um einen gebrauchsfertigen PMMA-Knochenzementteig daraus herzustellen. Die erfindungsgemäßen Vorrichtungen können mehrere Teile aufweisen, die in einfacher Weise miteinander verbunden und wieder voneinander getrennt werden können, und dabei eine zuverlässige und sichere Überführung einer Monomerflüssigkeit von einem Teil der Vorrichtung in das andere Teil der Vorrichtung erlauben.

Eine erste Ausführungsform betrifft eine Vorrichtung zur Herstellung eines Knochenzements, aufweisend einen Vorratsbehälter zur Aufnahme eines versiegelten Gefäßes mit einer Monomerflüssigkeit, und eine Kartusche zum Mischen von Knochenzement, wobei die Kartusche einen ersten Raum zur Aufnahme von Monomerflüssigkeit aus dem Vorratsbehälter und einen zweiten Raum zur Aufnahme eines Knochenzementpulvers aufweist, und wobei die Kartusche weiterhin ein Ventil aufweist, welches den ersten Raum mit dem zweiten Raum fluidleitend verbindet,
wobei die Vorrichtung ausgebildet und eingerichtet ist,
in einem ersten Schritt innerhalb des Vorratsbehälters eine Monomerflüssigkeit aus einem versiegelten Gefäß freizusetzen und an den ersten Raum der Kartusche abzugeben,
in einem zweiten Schritt die Monomerflüssigkeit innerhalb des ersten Raums zu halten, wobei ein Kontakt der Monomerflüssigkeit mit dem zweiten Raum der Kartusche vermieden wird, und in einem dritten Schritt die Monomerflüssigkeit aus dem ersten Raum der Kartusche in den zweiten Raum der Kartusche abzugeben.

Eine zweite Ausführungsform betrifft eine Vorrichtung gemäß der ersten Ausführungsform, wobei der Vorratsbehälter und die Kartusche als jeweils voneinander getrennte Elemente ausgestaltet sind, welche lösbar miteinander verbindbar sind.

Eine dritte Ausführungsform betrifft eine Vorrichtung gemäß der ersten oder zweiten Ausführungsform, wobei die Kartusche weiterhin einen verschiebbaren ersten Kolben aufweist, der mit dem Vorratsbehälter verbindbar ist.

Eine vierte Ausführungsform betrifft eine Vorrichtung gemäß einer der vorangehenden Ausführungsformen, wobei der erste Kolben eine erste Durchführung zur Abgabe einer Monomerflüssigkeit aus dem Vorratsbehälter den ersten Raum der Kartusche aufweist.

Eine fünfte Ausführungsform betrifft eine Vorrichtung gemäß einer der vorangehenden Ausführungsformen, wobei die Kartusche weiterhin einen verschiebbaren zweiten Kolben aufweist, welcher zwischen dem ersten Raum und dem zweiten Raum der Kartusche angeordnet ist.

Eine sechste Ausführungsform betrifft eine Vorrichtung gemäß einer der vorangehenden Ausführungsformen, wobei das Ventil in dem zweiten Kolben angeordnet ist.

Eine siebte Ausführungsform betrifft eine Vorrichtung gemäß einer der vorangehenden Ausführungsformen, wobei der zweite Kolben eine Vielzahl von Durchführungen aufweist, welche eine fluidleitende Verbindung zwischen dem Ventil und dem zweiten Raum der Kartusche ausbilden.

Eine achte Ausführungsform betrifft eine Vorrichtung gemäß der fünften, sechsten oder siebten Ausführungsform, wobei der zweite Kolben weiterhin eine fluidleitende poröse Schicht aufweist, welche eingerichtet ist, das Eindringen von Knochenzementpulver aus dem zweiten Raum der Kartusche in das Ventil und/oder ggf. die Durchführungen gemäß der siebten Ausführungsform zu verhindern.

Eine neunte Ausführungsform betrifft eine Vorrichtung gemäß einer der vorangehenden Ausführungsformen, wobei das Ventil ausgebildet und eingerichtet ist, in einem drucklosen Zustand des ersten Raums eine Monomerflüssigkeit vollständig in dem ersten Raum zu halten, und in einem druckbeaufschlagten Zustand des ersten Raums eine Monomerflüssigkeit an den zweiten Raum abzugeben.

Eine zehnte Ausführungsform betrifft eine Vorrichtung gemäß einer der vorangehenden Ausführungsformen, wobei das Ventil bevorzugt ein durch Druckbeaufschlagung zu öffnendes Ventil ist, wobei das Ventil weiter bevorzugt eine Feder und einen an der Feder gelagerten Dichtkörper aufweist, oder einen verklemmten, durch Druckbeaufschlagung lösbaren Dichtkörper aufweist, oder wobei das Ventil ein Bunsenventil ist.

Eine elfte Ausführungsform betrifft eine Vorrichtung gemäß einer der vorangehenden Ausführungsformen, welche ausgebildet und eingerichtet ist, durch Ausübung einer Kraft auf den ersten Raum und/oder den zweiten Raum Knochenzement aus der Vorrichtung nach außen abzugeben.

Eine zwölfte Ausführungsform betrifft eine Vorrichtung gemäß einer der vorangehenden Ausführungsformen, weiterhin aufweisend ein Auspresselement, wobei das Auspresselement ggf. zum Verschieben des ersten Kolben und/oder des zweiten Kolben ausgebildet und eingerichtet ist.

Eine dreizehnte Ausführungsform betrifft eine Vorrichtung gemäß einer der vorangehenden Ausführungsformen, wobei der Vorratsbehälter weiterhin ein Öffnungselement aufweist, das zum Brechen einer Glasampulle oder zum Öffnen eines Folienbeutels innerhalb des Vorratsbehälters ausgebildet und eingerichtet ist.

Eine vierzehnte Ausführungsform betrifft eine Vorrichtung gemäß der dreizehnten Ausführungsform, wobei die Vorrichtung ausgebildet und eingerichtet ist, eine Monomerflüssigkeit durch freien Gravitationsfluss aus dem Vorratsbehälter an den ersten Raum der Kartusche abzugeben.

Ein weiterer Aspekt betrifft die Verwendung einer Vorrichtung nach einem der vorangehenden Ansprüche zur Herstellung eines Knochenzements.

Ein weiterer Aspekt betrifft ein Verfahren zur Herstellung eines Knochenzements, aufweisend die nachfolgenden Schritte bis,
(a) Bereitstellen einer Vorrichtung gemäß einer der ersten bis vierzehnten Ausführungsformen,
(b) Freisetzen einer Monomerflüssigkeit aus einem versiegelten Gefäß innerhalb des Vorratsbehälters und Abgabe der Monomerflüssigkeit an den ersten Raum der Kartusche,
(c) Halten der Monomerflüssigkeit innerhalb des ersten Raums der Kartusche, wobei ein Kontakt der Monomerflüssigkeit mit dem zweiten Raum der Kartusche vermieden wird,
(d) Druckbeaufschlagen der Monomerflüssigkeit in dem ersten Raum und dadurch Öffnen des Ventils und Abgeben der Monomerflüssigkeit aus dem ersten Raum der Kartusche durch das Ventil in den zweiten Raum der Kartusche.

### Kurze Beschreibung der Zeichnungen

**Figur 1** zeigt einen Vorratsbehälter einer erfindungsgemäßen Vorrichtung.
**Figur 2** zeigt eine Kartusche einer erfindungsgemäßen Vorrichtung.
**Figur 3** zeigt eine erfindungsgemäße Vorrichtung, wobei ein Vorratsbehälter mit einer Kartusche in Kontakt gebracht ist.
**Figur 4** zeigt eine erfindungsgemäße Vorrichtung, wobei eine Monomerflüssigkeit aus dem Vorratsbehälter in einen ersten Raum der Kartusche überführt ist.
**Figur 5** zeigt eine Kartusche mit einem ersten Kolben, der durch einen Stopfen verschlossen ist.
**Figur 6** zeigt eine Kartusche, die im Eingriff mit einer Auspressstange steht.
**Figur 7** zeigt eine Kartusche, bei welcher ein erster Kolben mithilfe einer Auspressstange in Richtung eines zweiten Kolbens bewegt ist.
**Figur 8** zeigt eine Kartusche, bei welcher ein erster Kolben mithilfe einer Auspressstange gegen einen zweiten Kolben gedrückt ist.
**Figur 9** zeigt eine Kartusche mit fertig gemischtem Knochenzement, wobei die Kartusche durch Entfernen eines Deckels geöffnet ist.
**Figur 10** zeigt eine Kartusche mit fertig gemischtem Knochenzement, wobei die Kartusche einen Schnorchel zum Ausbringen von Knochenzement aufweist.

### AUSFÜHRLICHE BESCHREIBUNG

Zu den hierin beschriebenen Ausführungsformen, deren Elemente ein bestimmtes Merkmal (z.B. ein Material) "aufweisen", "enthalten", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "enthalten", "enthaltend", "aufweisen" oder "aufweisend" verwendet.

"Operativ verbunden" oder "operativ verbindbar", bedeutet hierin, dass zwei betreffende Elemente eine funktionelle Beziehung zueinander aufweisen. Beispielsweise kann ein erstes Element eingerichtet sein, ein zweites Element durch eine solche operative Verbindung zu steuern oder zu bewegen. Der Begriff "steuern" umfasst hierin auch die Sperrung oder Freigabe einer Funktion, beispielsweise das Ermöglichen oder die Einschränkung der Bewegung oder sonstigen Funktion eines Elements.

Wenn in einer Ausführungsform ein Element mit dem Singular bezeichnet ist, wird hierzu stets ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse, Vorrichtungen, Kits und Verwendungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Eine erste Ausführungsform betrifft eine Vorrichtung zur Herstellung eines Knochenzements, aufweisend einen Vorratsbehälter zur Aufnahme eines versiegelten Gefäßes mit einer Monomerflüssigkeit, und eine Kartusche zum Mischen von Knochenzement, wobei die Kartusche einen ersten Raum zur Aufnahme von Monomerflüssigkeit aus dem Vorratsbehälter und einen zweiten Raum zur Aufnahme eines Knochenzementpulvers aufweist, und wobei die Kartusche weiterhin ein Ventil aufweist, welches den ersten Raum mit dem zweiten Raum fluidleitend verbindet, wobei die Vorrichtung ausgebildet und eingerichtet ist,
in einem ersten Schritt innerhalb des Vorratsbehälters eine Monomerflüssigkeit aus einem versiegelten Gefäß freizusetzen und an den ersten Raum der Kartusche abzugeben,
in einem zweiten Schritt die Monomerflüssigkeit innerhalb des ersten Raums zu halten, wobei ein Kontakt der Monomerflüssigkeit mit dem zweiten Raum der Kartusche vermieden wird, und in einem dritten Schritt die Monomerflüssigkeit aus dem ersten Raum der Kartusche in den zweiten Raum der Kartusche abzugeben.

Für das Aufbrechen eines versiegelten Gefäßes innerhalb der Vorrichtung ist häufig ein erhöhter Kraftaufwand erforderlich. Die erfindungsgemäßen Vorrichtungen erlauben es, das Aufbrechen eines versiegelten Gefäßes mit einer Monomerflüssigkeit zeitlich von dem Auspressen des in der Vorrichtung gemischten Knochenzements zu entkoppeln. Hierdurch kann beispielsweise eine erste Person, z.B. ein Assistent, ein versiegeltes Gefäß mit einer Monomerflüssigkeit innerhalb der Vorrichtung öffnen und in den ersten Raum der Kartusche transferieren, und eine zweite Person, z.B. ein behandelnder Arzt, kann die in dem ersten Raum der Kartusche zwischengelagerte Monomerflüssigkeit in den zweiten Raum der Kartusche drücken, um dadurch einen gebrauchsfertigen Knochenzement herzustellen. Dieser Knochenzement kann in einem weiteren Schritt aus der Kartusche gedrückt werden. Hierdurch muss die zweite Person keine Kraft aufwenden, um das versiegelte Gefäß mit Monomerflüssigkeit aufzubrechen und das geborstene Gefäß zu komprimieren.

Die Vorrichtung ist bevorzugt zur Herstellung eines Polymethylmethacrylat-Knochenzements geeignet. In einer Ausführungsform umfasst die Vorrichtung eine Monomerflüssigkeit und ein Knochenzementpulver.

Polymethylmethacrylat-Knochenzemente umfassen üblicherweise eine flüssige Monomerkomponente, hierin auch als "Monomerflüssigkeit" bezeichnet, und eine Pulverkomponente, hierin auch als "Knochenzementpulver" bezeichnet. Die Monomerflüssigkeit kann beispielsweise das Monomer Methylmethacrylat und gegebenenfalls einen darin gelösten Aktivator umfassen. Ein Beispiel für einen geeigneten Aktivator ist N,N-Dimethyl-p-toluidin. Das Knochenzementpulver weist bevorzugt ein Polymer auf. Geeignete Polymere sind durch Polymerisation aus Methylmethacrylat und Comonomeren herstellbar. Beispiele für geeignete Comonomere umfassen Styren und/oder Methylacrylat.

Unter "Knochenzement" werden hierin Gemische aus einer solchen Monomerflüssigkeit und einem solchen Knochenzementpulver verstanden. Der Begriff "Knochenzement" umfasst hierbei insbesondere frisch hergestellte Mischungen dieser beiden Komponenten, welche eine pastöse Konsistenz aufweisen, und zu einem harten Polymer aushärten können.

Die Vorrichtung ist zur Aufnahme eines versiegelten Gefäßes mit einer Monomerflüssigkeit geeignet. In einigen Ausführungsformen umfasst die Vorrichtung ein solches Gefäß. In einer Ausführungsform ist das Gefäß eine Glasampulle. In einer Ausführungsform ist das Gefäß ein Folienbeutel. Bevorzugt umfasst der Folienbeutel ein Verbundmaterial. Geeignete Verbundmaterialien umfassen einen Kunststoff und/oder ein Metall, wobei das Metall bevorzugt Aluminium umfasst. Unter "versiegelt" wird hierin insbesondere verstanden, dass das Gefäß den Kontakt einer darin enthaltenen Monomerflüssigkeit mit der umgebenden Raumluft verhindert.

Die erfindungsgemäße Vorrichtung weist einen Vorratsbehälter und eine Kartusche auf. Diese beiden Elemente können miteinander verbindbar sein. Bevorzugt sind der Vorratsbehälter und die Kartusche lösbar miteinander verbindbar. Dies ermöglicht eine Bereitstellung der erfindungsgemäßen Vorrichtung in einer Form, in welcher der Vorratsbehälter und die Kartusche zunächst getrennt voneinander vorliegen. Eine solche Bereitstellung in Form zweier getrennter Komponenten bietet Vorteile bezüglich Herstellung, Sterilisierung, Lagerung und Transport.

Dementsprechend kann die Vorrichtung als ein Kit bereitgestellt werden, welches mehrere Teile enthält. Das Kit kann als erstes Teil einen Vorratsbehälter und als zweites Teil eine Kartusche aufweisen, wie hierin ausführlich beschrieben.

Die Kartusche weist einen ersten Raum zur Aufnahme von Monomerflüssigkeit aus dem Vorratsbehälter und einen zweiten Raum zur Aufnahme eines Knochenzementpulvers auf.

Der erste Raum der Kartusche kann zur Zwischenlagerung einer Monomerflüssigkeit aus dem Vorratsbehälter dienen.

Die Kartusche kann mit einem Knochenzementpulver vorbefüllt sein. Der zweite Raum der Kartusche kann ein Knochenzementpulver aufweisen. Bevorzugt kann der zweite Raum der Kartusche ein verdichtetes Knochenzementpulver aufweisen. Dies bedeutet, dass der zweite Raum der Kartusche im Wesentlichen vollständig mit einem Knochenzementpulver befüllt ist, und keine ungefüllten Hohlräume in dem zweiten Raum der Kartusche vorhanden sind. Dies ermöglicht eine homogene Vermischung von Monomerflüssigkeit und Knochenzementpulver, ohne dass es hierzu eines mechanischen Rührelements bedarf. In einer Ausführungsform weist die Vorrichtung daher kein Rührelement auf. Ein Beispiel für ein Rührelement ist ein Mischstab, der ein oder mehrere Mischblätter aufweist. In einer Ausführungsform ist die Vorrichtung eingerichtet, eine Monomerflüssigkeit und ein Knochenzementpulver durch bloßes Einspritzen der Monomerflüssigkeit in ein verdichtetes Knochenzementpulver zu mischen. Dies bedeutet, dass ohne einen mechanischen Rührvorgang ein Knochenzement aus der Monomerflüssigkeit und dem Knochenzementpulver gebildet wird.

Der zweite Raum weist bevorzugt einen Hohlraum auf, welcher ein Volumen aufweist, welches das Volumen der aufzunehmenden Monomerflüssigkeit um mindestens 20 %, weiter bevorzugt mindestens 30 % übersteigt.

Die Vorrichtung ist eingerichtet, in einem ersten Schritt innerhalb des Vorratsbehälters eine Monomerflüssigkeit aus einem versiegelten Gefäß freizusetzen und an den ersten Raum der Kartusche abzugeben. Hierzu kann der Vorratsbehälter ein Mittel zur Öffnung eines solchen Gefäßes enthalten, welches hierin auch als "Öffnungselement" bezeichnet ist. In einigen Ausführungsformen weist der Vorratsbehälter weiterhin ein Mittel zur Öffnung eines Folienbeutels oder einer Glasampulle innerhalb des Vorratsbehälters auf. Ein Beispiel für ein Mittel zur Öffnung eines Folienbeutels ist ein Dorn, welcher zum Durchstechen eines Folienbeutels geeignet ist. Ein solcher Dorn kann beispielsweise aus einem starren Kunststoff oder aus Metall ausgebildet sein. Ein Beispiel für ein Mittel zur Öffnung einer Glasampulle ist ein Vorsprung, gegen den der Kopf einer Glasampulle gedrückt werden kann, um sie aufzubrechen. Ein Mittel zur Öffnung einer Glasampulle kann ein flexibles Gehäuseteil aufweisen, so dass die Glasampulle von außen mit der Hand gebrochen werden kann, indem das flexible Gehäuseteil bewegt und/oder verformt wird. Der Vorratsbehälter kann hierzu ein flexibles Gehäuseteil und ein starres Gehäuseteil aufweisen, wobei das flexible Gehäuseteil gegenüber dem starren Gehäuseteil bewegt und/oder verformt wird. Ein Mittel zur Öffnung einer Glasampulle kann auch ein beweglich angeordnetes Element wie z.B. einen Kolben aufweisen, welches die Glasampulle gegen ein hartes Gehäuseteil, z.B. einen Vorsprung, drücken und dadurch aufbrechen kann. Beispiele für geeignete Öffnungselemente, insbesondere zur Öffnung eines Folienbeutels oder einer Glasampulle, sind weiterhin in DE19532015A1, WO9718031A1, EP2404864B1 und WO2010012114A1 offenbart.

Der Vorratsbehälter kann weiterhin einen Träger zur Aufnahme eines Gefäßes mit einer Monomerflüssigkeit aufweisen. Der Träger ist bevorzugt ausgebildet und eingerichtet, das Gefäß innerhalb des Vorratsbehälters in einer definierten Position zu halten. Der Träger kann beispielsweise eine ringförmige Struktur aufweisen, welche ausgebildet ist, den Hals einer Glasampulle zu umgeben. Eine solche ringförmige Struktur kann beispielsweise als offene oder geschlossene ringförmige Strebe ausgestaltet sein, oder kann als Öffnung in einer Oberfläche des Trägers ausgestaltet sein.

Der Träger kann ausgebildet und eingerichtet sein, dass Gefäß gegen das Öffnungselement zu bewegen. Der Träger kann ausgebildet und eingerichtet sein, in Zusammenwirkung mit dem Öffnungselement das Gefäß zu öffnen. Beispielsweise kann der Vorratsbehälter einen Träger aufweisen, der beweglich in dem Vorratsbehälter angeordnet ist. Der Träger kann ausgebildet und eingerichtet sein, das Gefäß gegen ein Öffnungselement, wie z.B. gegen einen Vorsprung, einen Dorn oder eine Schneidvorrichtung, zu bewegen, um das Gefäß dadurch zu öffnen.

Der Vorratsbehälter kann weiterhin einen Filter aufweisen, der ausgebildet und eingerichtet ist, Bruchstücke des Gefäßes in dem Vorratsbehälter zurückzuhalten. Der Filter kann beispielsweise als Sieb ausgestaltet sein, in welchem Glasbruchstücke einer aufgebrochenen Glasampulle zurückgehalten werden können. Ein solches Sieb kann als Metalldrahtnetz oder Polymernetz ausgestaltet sein. Das Sieb kann auch eine Lochscheibe aus Metall, Glas, Keramik oder Kunststoff aufweisen.

Der Vorratsbehälter kann ein erstes Ende und ein zweites Ende aufweisen. Der Vorratsbehälter ist bevorzugt ausgebildet und eingerichtet, an dem zweiten Ende des Vorratsbehälters mit der Kartusche verbindbar zu sein, bevorzugt lösbar verbindbar zu sein.

Der Vorratsbehälter kann weiterhin einen Stutzen aufweisen. Der Stutzen kann zur Abgabe von Monomerflüssigkeit aus dem Vorratsbehälter an die Kartusche ausgebildet sein. Der Stutzen ist bevorzugt am zweiten Ende des Vorratsbehälters angeordnet. Der Stutzen kann bevorzugt eine konische Form aufweisen. In einer Ausführungsform weist der Stutzen eine Form auf, die sich nach außen hin verjüngt. Somit kann der Vorratsbehälter einen trichterförmigen Auslass aufweisen, welcher am zweiten Ende des Vorratsbehälters durch den Stutzen gebildet ist. Der Stutzen kann mittig am zweiten Ende des Vorratsbehälters angeordnet sein. Bei einem zylinderförmigen Vorratsbehälter entspricht dies einer Anordnung des Stutzens in Verlängerung der Längsmittelachse des Zylinders. Der Vorratsbehälter kann weiterhin ein erstes Verbindungselement aufweisen, das zur Verbindung mit einem zweiten Verbindungselement der Kartusche ausgebildet und eingerichtet ist. Das erste Verbindungselement und das zweite Verbindungselement können miteinander eine kraftschlüssige und/oder formschlüssige Verbindung ausbilden. Beispiele für eine solche Verbindung umfassen einen Schraubverschluss mit einem Gewinde, oder einen Bajonettverschluss. Das erste Verbindungselement und das zweite Verbindungselement sind bevorzugt lösbar miteinander verbindbar. Das erste Verbindungselement ist bevorzugt an dem zweiten Ende des Vorratsbehälters angebracht.

Zur sicheren Verbindung können das erste Verbindungselement und/oder das zweite Verbindungselement weiterhin einen Rastmechanismus aufweisen. In einer Ausführungsform weist der Rastmechanismus beispielsweise ein oder mehrere bewegliche Rastelemente auf, die ausgebildet und eingerichtet sind, sich radial zu bewegen, wenn der Vorratsbehälter in Kontakt mit der Kartusche gebracht wird, und in entsprechende Rillen oder Vertiefungen an der Kartusche eingreifen. Der Rastmechanismus kann bevorzugt federbelastetet sein, um ein Eingreifen der Rastelemente in ihr Gegenstück zu ermöglichen.

Der Rastmechanismus kann eine Entriegelung aufweisen, welche zum Lösen der Rastelemente ausgebildet und eingerichtet ist.

Die erfindungsgemäße Vorrichtung ist in einer Ausführungsform derart ausgebildet, dass der Vorratsbehälter und die Kartusche derart miteinander verbindbar sind, dass ihre jeweiligen Längsmittelachsen miteinander eine gemeinsame Linie bilden. Hierdurch ergibt sich eine insgesamt lineare Anordnung von Vorratsbehälter und Kartusche. In einer Ausführungsform bildet die Längsmittelachse des Vorratsbehälters mit der Längsmittelachse der Kartusche einen Winkel, welcher 0° bis 50° beträgt, beispielsweise 0° bis 40°, 0° bis 30°, 0° bis 20° oder 0° bis 10°. In einer Ausführungsform beträgt dieser Winkel 0°.

Der Vorratsbehälter kann weiterhin ein Bedienelement aufweisen. Bevorzugt ist das Bedienelement ausgebildet und eingerichtet, das Gefäß zu öffnen. Besonders bevorzugt ist das Bedienelement ausgebildet und eingerichtet, in Zusammenwirkung mit dem hierin beschriebenen Öffnungselement des Gefäßes das Gefäß zu öffnen. Das Bedienelement kann beispielsweise als Griffelement ausgestaltet sein, welches eine Bewegung des Gefäßes in Richtung des Öffnungselements erlaubt. Beispielsweise kann ein Benutzer der Vorrichtung über das Bedienelement eine Kraft auf das Gefäß ausüben, um es gegen einen Vorsprung, Dorn oder ein Schneidelement des Vorratsbehälters zu drücken und es dadurch zu öffnen.

Der Vorratsbehälter weist bevorzugt eine im wesentlichen zylindrische Form auf. Insbesondere kann die Gesamtaußenoberfläche des Vorratsbehälters eine im wesentlichen zylindrische Form aufweisen.

Die Kartusche weist einen ersten Raum, einen zweiten Raum und ein Ventil auf, welches den ersten Raum mit dem zweiten Raum fluidleitend verbindet. Bevorzugt bildet das Ventil die einzige fluidleitende Verbindung zwischen dem ersten Raum und dem zweiten Raum. Dies bedeutet, dass Monomerflüssigkeit zwingend durch das Ventil geleitet werden muss, um aus dem ersten Raum in den zweiten Raum zu gelangen.

Das Ventil ist bevorzugt unidirektional. Dies bedeutet, dass das Ventil ausgebildet und eingerichtet ist, eine Monomerflüssigkeit nur in einer Richtung passieren zu lassen. Bevorzugt ist das Ventil ausgebildet und eingerichtet, Monomerflüssigkeit aus dem ersten Raum in den zweiten Raum der Kartusche zu leiten. Bevorzugt ist das Ventil weiterhin ausgebildet und eingerichtet, ein Fließen von Monomerflüssigkeit aus dem zweiten Raum in den ersten Raum der Kartusche zu verhindern.

Das Ventil ist bevorzugt ein druckabhängiges Ventil. Ein solches Ventil ist ausgebildet und eingerichtet, sich durch Druckbeaufschlagung zu öffnen. In einer Ausführungsform ist das Ventil ausgebildet und eingerichtet, in einem drucklosen Zustand des ersten Raums eine Monomerflüssigkeit vollständig in dem ersten Raum zu halten, und in einem druckbeaufschlagten Zustand des ersten Raums eine Monomerflüssigkeit an den zweiten Raum abzugeben. Das Ventil kann ausgebildet und eingerichtet sein, sich oberhalb eines vorbestimmten Grenzdrucks zu öffnen. Ein solcher Grenzdruck ist erfindungsgemäß als Druckdifferenz definiert, welche an den beiden Seiten des Ventils anliegt. Zweckmäßig kann dieser Grenzdruck als Druckdifferenz des ersten Raums im Vergleich zum zweiten Raum der Kartusche mit einem Manometer bei einer Temperatur von 25 °C bestimmt werden, während der erste Raum und der zweite Raum mit gewöhnlicher Raumluft gefüllt sind. Dieser Grenzdruck kann beispielsweise im Bereich von 0,5 bar bis 1,5 bar liegen. In einer Ausführungsform liegt der Grenzdruck bei ungefähr 1 bar (10^5 Pa), beispielsweise 0,8 bis 1,2 bar oder 0,9 bis 1,1 bar. In einer Ausführungsform liegt der Grenzdruck bei ungefähr 0,5 bar (0,5 × 10^5 Pa), beispielsweise 0,3 bis 0,7 bar oder 0,4 bis 0,6 bar.

Das Ventil ist in einer Ausführungsform als Rückschlagventil ausgestaltet. Das Ventil kann eine Feder und einen an der Feder gelagerten Dichtkörper aufweisen.

Das Ventil kann einen verklemmten Dichtkörper aufweisen, der durch Druckbeaufschlagung lösbar ist. Der Dichtkörper des Ventils kann ausgebildet und eingerichtet sein, in einem drucklosen Zustand des Ventils das Ventil abzudichten und dadurch geschlossen zu halten. Der Dichtkörper des Ventils kann ausgebildet und eingerichtet sein, in einem druckbeaufschlagten Zustand des Ventils das Ventil freizugeben, um eine fluidleitende Verbindung durch das Ventil herzustellen. Der Dichtkörper kann beispielsweise eine kugelförmige, ovoide oder zylindrische Form aufweisen. Der Dichtkörper kann einen Kunststoff, eine Keramik oder ein Metall aufweisen, oder daraus bestehen. Der Kunststoff ist bevorzugt ein thermoplastischer Kunststoff. Das Ventil kann einen Kanal aufweisen, in welchem der Dichtkörper angeordnet ist. In einer Ausführungsform weist der Kanal einen Innendurchmesser auf, der mindestens das 1,1-fache, bevorzugt mindestens das 2-fache eines Durchmessers des Dichtkörpers aufweist. Dies ermöglicht es der Monomerflüssigkeit, den Dichtkörper zu passieren, wenn dieser aus dem verklemmten Zustand gelöst ist.

Das Ventil kann eingerichtet sein, aus einem geöffneten Zustand des Ventils selbsttätig in einen geschlossenen Zustand des Ventils überzugehen, wenn das Ventil von einem druckbeaufschlagten Zustand in einen nicht druckbeaufschlagten Zustand übergeht. Hierdurch kann beispielsweise das Ventil geöffnet werden, wenn innerhalb des ersten Raums der Kartusche eine Monomerflüssigkeit mit Druck beaufschlagt wird, um die Monomerflüssigkeit durch das Ventil in den zweiten Raum zu überführen, und das Ventil wieder geschlossen werden, nachdem die Monomerflüssigkeit in den zweiten Raum überführt wurde, und im ersten Raum der Kartusche keine Überdruck mehr Verhältnis zum zweiten Raum der Kartusche vorliegt.

Die Vorrichtung kann zur Aufnahme eines definierten Volumens einer Monomerflüssigkeit ausgebildet und eingerichtet sein. Beispielsweise kann die Vorrichtung einen Träger enthalten, der für handelsübliche Glasampullen oder Folienbeutel mit einem bestimmten Volumen einer Monomerflüssigkeit angepasst ist. In diesem Zusammenhang kann der erste Raum der Kartusche ein Innenvolumen aufweisen, welches dieses definierte Volumen einer Monomerflüssigkeit übersteigt. Beispielsweise kann der erste Raum der Kartusche ein Innenvolumen aufweisen, welches das definierte Volumen einer Monomerflüssigkeit um mindestens 20 % oder bevorzugt um mindestens 30 % übersteigt.

Das Ventil ist in einer Ausführungsform als Bunsenventil ausgestaltet. Erfindungsgemäß weist ein Bunsenventil ein gummielastisches Material mit einer Öffnung auf, welche in einem drucklosen Zustand des Ventils geschlossen ist, und ausgebildet und eingerichtet ist, sich in einem druckbeaufschlagten Zustand des Ventils zu öffnen. Beispielsweise kann ein Bunsenventil eine Gummischeibe mit einem Schlitz aufweisen. In einer Ausführungsform weist dieser Schlitz eine gerade, gebogene, sternförmige, kreuzförmige oder hufeisenförmige Form auf. In einer Ausführungsform ist das Bunsenventil eingerichtet, sich durch eine elastische Rückstellkraft des gummielastischen Materials reversibel zu öffnen und schließen. In einer Ausführungsform weist das Bunsenventil Schlitzwände auf, welche sich im geschlossenen Zustand eines Abgabeventils berühren, und sich beim Öffnen eines Abgabeventils voneinander entfernen. Die Schlitzwände sind bevorzugt rechtwinklig zu einer Oberfläche des gummielastischen Materials angeordnet.

Das gummielastische Material ist bevorzugt ein Polymer, insbesondere ein Elastomer. Bevorzugt umfasst das gummielastische Material ein medizinisch verträgliches Elastomer, oder besteht daraus. Beispiele für medizinisch verträgliche Elastomere umfassen Silikonelastomere, thermoplastische Elastomere (TPEs), Polyisopren, Butylkautschuk, Nitrilkautschuk, Ethylen-Propylen-Dien-Monomer (EPDM), Chloropren-Kautschuk, Fluroelastomere, Perfluorelastomere und Polyacrylatelastomere. Beispiele für Silikonelastomere umfassen Polydimethylsiloxan (PDMS) und flüssiger Silikonkautschuk (LSR).

Beispiele für thermoplastische Elastomere (TPEs) umfassen Styrol-Block-Copolymere (SBCs) wie Styrol-Ethylen-Butylen-Styrol (SEBS), thermoplastische Polyurethane (TPU) und thermoplastische Copolyester (TCE). Beispiele für Polyisopren umfassen natürlichen Gummi und synthetischen Polyisopren. Beispiele für Butylkautschuk umfassen Brombutylkautschuk (BIIR) und Chlorbutylkautschuk (CIIR). Ein bevorzugtes Polyurethan ist ein Polyetherurethan. Polyetherurethane sind durch Polyadditionsreaktion eines Polyetherpolyols mit einem Diisocyanat herstellbar.

In einer Ausführungsform weist das gummielastische Material ein thermoplastisches Elastomer auf. In einer Ausführungsform besteht das gummielastische Material aus einem thermoplastischen Elastomer. In einer Ausführungsform weist das gummielastische Material ein Polyetherurethan oder Ethylen-Propylen-dien-Kautschuk auf. In einer Ausführungsform umfasst das gummielastische Material ein Polyetherurethan. In einer Ausführungsform besteht das gummielastische Material aus Polyetherurethan.

In einer Ausführungsform weist das gummielastische Material nur ein einziges Elastomer auf, d. h. dem gummielastischen Material ist kein zweites Elastomer beigemischt. In einer Ausführungsform weist das gummielastische Material mindestens zwei unterschiedliche Materialien, beispielsweise zwei unterschiedliche Elastomere, auf.

Das gummielastische Material kann in einer Ausführungsform einen Young-Elastizitätsmodul von 1,2 × 10^7 Pa bis 2,1 × 10^7 Pa aufweisen, beispielsweise 1,3 × 10^7 Pa bis 2,0 × 10^7 Pa, 1,4 × 10^7 Pa bis 1,9 × 10^7 Pa, 1,5 × 10^7 Pa bis 1,8 × 10^7 Pa, oder 1,5 × 10^7 Pa bis 1,7 × 10^7 Pa. In einer Ausführungsform kann das gummielastische Material einen Young-Elastizitätsmodul von ungefähr 1,6 × 10^7 Pa aufweisen. In einer Ausführungsform kann das gummielastische Material einen Young-Elastizitätsmodul von 2000 bis 2500 psi aufweisen. Letzteres entspricht ungefähr 1,4 × 10^7 Pa bis 1,7 × 10^7 Pa.

Der Young-Elastizitätsmodul kann nach ASTM D412 bestimmt werden.

Das gummielastische Material ist bevorzugt mithilfe gängiger Sterilisationsverfahren sterilisierbar, d. h. im Rahmen dieser Verfahren beständig gegenüber UV-Strahlung, Gammastrahlung und/oder Behandlung mit Ethylenoxid.

Das gummielastische Material ist bevorzugt mithilfe fachüblicher Extrusionsverfahren und/oder Spritzgussverfahren formbar.

In einer Ausführungsform weist das gummielastische Material eine Shore-A-Härte im Bereich von 30 bis 80 auf. In einer Ausführungsform weist das gummielastische Material einer Shore-A-Härte von 40 bis 70, oder 50 bis 60, beispielsweise ungefähr 55, auf. Die Shore-A-Härte wird nach ASTM D2240 bestimmt. Elastomere in diesem Bereich der Shore A-Härte haben eine sehr gute Rückstellkraft und eignen sich sehr gut zur Herstellung einer hierin beschriebenen gummielastischen Scheibe oder gummielastischen Beschichtung als Teil eines Abgabeventils. Schlitze in solchen Elastomeren verschließen sich bei Abfall des darauf einwirkenden Drucks selbstständig und schnell. In einer Ausführungsform weist das gummielastische Material eine Shore-A-Härte im Bereich von 75 bis 95 auf.

In einer Ausführungsform weist die Kartusche weiterhin einen verschiebbaren ersten Kolben auf. Bevorzugt begrenzt der erste Kolben den ersten Raum der Kartusche nach außen. Der erste Kolben kann scheibenförmig ausgebildet sein. Der erste Kolben kann formschlüssig in der Kartusche angebracht sein. Beispielsweise kann der erste Kolben in einem zylindrischen Innenraum der Kartusche verklemmt sein. In einer Ausführungsform greift der erste Kolben in ein Rastelement, beispielsweise einen Vorsprung oder eine Rille, an der Kartusche ein. In einer Ausführungsform ist der erste Kolben mit dem Vorratsbehälter verbindbar. In einer Ausführungsform ist der erste Kolben durch Druck des Vorratsbehälters gegen den ersten Kolben verschiebbar. Dies ermöglicht es einem Benutzer der Vorrichtung, durch Inkontaktbringen des Vorratsbehälters mit der Kartusche zunächst eine Monomerflüssigkeit in den ersten Raum der Kartusche zu überführen, und danach durch Bewegung des ersten Kolbens die Monomerflüssigkeit aus dem ersten Raum der Kartusche in den zweiten Raum der Kartusche zu überführen.

Der erste Kolben kann einen Außendurchmesser aufweisen, der einen Innendurchmesser der Kartusche übersteigt. Beispielsweise kann der Außendurchmesser des ersten Kolbens mindestens das 1,1-fache des Innendurchmessers der Kartusche betragen. In einer Ausführungsform ist der Außendurchmesser des ersten Kolbens mindestens 0,1 mm größer als der Innendurchmesser der Kartusche. In einer Ausführungsform ist der Außendurchmesser des ersten Kolbens um 0,1 bis 1,0 mm größer als der Innendurchmesser der Kartusche. Diese beiden Maße beziehen sich jeweils auf einen Gleichgewichtszustand des Kolbens und der Kartusche, in denen keine Kraft auf sie einwirkt, welche zu einer Verformung führen könnte. In einer Ausführungsform weist die Kartusche einen zylindrischen Innenraum auf, und der Innendurchmesser der Kartusche ist als lichte Weite dieses Innenraums definiert. In einer Ausführungsform weist der erste Kolben eine zylindrische Form auf, und der Außendurchmesser des ersten Kolbens ist als Außendurchmesser dieser zylindrischen Form definiert. Aufgrund eines solchen Übermaßes des ersten Kolbens kann dieser in der Kartusche verklemmt werden, so dass eine Verschiebung des ersten Kolbens innerhalb der Kartusche nur oberhalb einer definierten Kraft erfolgen kann, die auf den ersten Kolben einwirkt.

In einer Ausführungsform weist der erste Kolben eine erste Durchführung zur Abgabe einer Monomerflüssigkeit aus dem Vorratsbehälter an den ersten Raum der Kartusche auf. Die erste Durchführung kann beispielsweise eine zylindrische oder konische Form aufweisen. Die erste Durchführung kann ausgebildet und eingerichtet sein, eine Verbindung, bevorzugt eine formschlüssige Verbindung, mit einem Stutzen des Vorratsbehälters auszubilden.

Die erste Durchführung kann einen Vorsprung und/oder eine Verjüngung aufweisen, sodass der Stutzen nur bis zu einer gewünschten Position in die erste Durchführung einbringbar ist.

Die erste Durchführung kann einen Verschluss aufweisen. In einer Ausführungsform ist der Verschluss als Stopfen ausgebildet. Der Stopfen kann fest mit dem ersten Kolben verbunden sein, oder kann als separates Teil ausgebildet sein, das nicht fest mit dem ersten Kolben verbunden ist. Der Verschluss kann eine Dichtung aufweisen. Beispielsweise kann der Verschluss eine umlaufende Nut aufweisen, in der ein Dichtungsring angeordnet ist. Der Stopfen kann eine konische Form aufweisen. In einer Ausführungsform weist der Verschluss eine ausreichende Länge auf, um sich entlang der gesamten Länge der Durchführung durch den ersten Kolben hindurchzuerstrecken. In einer Ausführungsform schließt der Verschluss mit einer ersten Außenfläche des ersten Kolbens und/oder mit einer zweiten Außenfläche des ersten Kolbens ab. Bevorzugt sind die erste Außenfläche und die zweite Außenfläche an jeweils gegenüberliegenden Enden des ersten Kolbens angeordnet. Bevorzugt ist die erste Außenfläche des ersten Kolbens zu einer Außenseite der Kartusche weisend angeordnet. Bevorzugt ist die zweite Außenfläche des zweiten Kolbens zu einer Innenseite der Kartusche weisend angeordnet.

In einer Ausführungsform ist die Vorrichtung ausgebildet und eingerichtet, eine Monomerflüssigkeit aus dem Vorratsbehälter durch freien Gravitationsfluss an die Kartusche abzugeben. Dies bedeutet insbesondere, dass es keiner Druckbeaufschlagung des Vorratsbehälters bedarf, um die Monomerflüssigkeit aus dem Vorratsbehälter abzugeben.

In einer weiteren Ausführungsform weist die Kartusche weiterhin einen verschiebbaren zweiten Kolben auf, welcher zwischen dem ersten Raum und dem zweiten Raum der Kartusche angeordnet ist.

Bevorzugt begrenzt der zweite Kolben den zweiten Raum der Kartusche nach außen. Der zweite Kolben kann beispielsweise scheibenförmig ausgebildet sein. Der zweite Kolben kann formschlüssig in der Kartusche angeordnet sein. Beispielsweise kann der zweite Kolben in einem zylindrischen Innenraum der Kartusche verklemmt sein. In einer Ausführungsform greift der Kolben in ein Rastelement, beispielsweise einen Vorsprung oder eine Rille, an der Kartusche ein. In einer Ausführungsform ist der zweite Kolben durch Druck des ersten Kolbens gegen den zweiten Kolben verschiebbar. Dies ermöglicht es einem Benutzer der Vorrichtung, den in der Vorrichtung gemischten Knochenzement aus der Vorrichtung abzugeben.

Der zweite Kolben kann einen Außendurchmesser aufweisen, der einen Innendurchmesser der Kartusche übersteigt. Beispielsweise kann der Außendurchmesser des zweiten Kolbens mindestens das 1,1-fache des Innendurchmessers der Kartusche betragen. In einer Ausführungsform ist der Außendurchmesser des zweiten Kolbens mindestens 0,1 mm größer als der Innendurchmesser der Kartusche. In einer Ausführungsform ist der Außendurchmesser des zweiten Kolbens um 0,1 bis 1,0 mm größer als der Innendurchmesser der Kartusche. Diese beiden Maße beziehen sich jeweils auf einen Gleichgewichtszustand des Kolbens und der Kartusche, in denen keine Kraft auf sie einwirkt, welche zu einer Verformung führen könnte. In einer Ausführungsform weist die Kartusche einen zylindrischen Innenraum auf, und der Innendurchmesser der Kartusche ist als lichte Weite dieses Innenraums definiert. In einer Ausführungsform weist der zweite Kolben eine zylindrische Form auf, und der Außendurchmesser des zweiten Kolbens ist als Durchmesser dieser zylindrischen Form definiert. Aufgrund eines solchen Übermaßes des zweiten Kolbens kann dieser in der Kartusche verklemmt werden, so dass eine Verschiebung des zweiten Kolbens innerhalb der Kartusche nur oberhalb einer definierten Kraft erfolgen kann, die auf den zweiten Kolben einwirkt.

In einer Ausführungsform weisen der erste Kolben und der zweite Kolben jeweils denselben Außendurchmesser auf. In einer Ausführungsform weisen der erste Kolben und der zweite Kolben im Wesentlichen dieselbe Außenform auf. Hiermit ist jeweils die Grundform des ersten Kolben bzw. zweiten Kolben gemeint, d. h. darin enthaltene Elemente wie z.B. eine Durchführung oder ein Ventil bleiben hierbei außer Betracht. Beispielsweise können der erste Kolben und der zweite Kolben jeweils eine im wesentlichen scheibenförmige Außenform aufweisen.

Bevorzugt ist das Ventil, welches den ersten Raum der Kartusche mit dem zweiten Raum der Kartusche fluidleitend verbindet, in dem zweiten Kolben angeordnet.

Der zweite Kolben kann weiterhin eine Vielzahl von Durchführungen aufweisen. Diese Durchführungen können eine fluidleitende Verbindung zwischen dem Ventil und dem zweiten Raum der Kartusche ausbilden. Diese Durchführungen können ausgebildet und eingerichtet sein, eine Monomerflüssigkeit, die aus dem ersten Raum in den zweiten Raum abgegeben wird, an verschiedenen Positionen in den zweiten Raum zu leiten. Hierdurch kann die Monomerflüssigkeit besser innerhalb des zweiten Raums verteilt werden, und sich dadurch gleichmäßiger mit einem darin enthaltenen Knochenzementpulver vermischen.

In einer Ausführungsform weist der zweite Kolben weiterhin eine fluidleitende poröse Schicht auf. Diese Schicht ist bevorzugt eingerichtet, das Eindringen von Knochenzementpulver aus dem zweiten Raum der Kartusche in das Ventil und/oder die Durchführungen des zweiten Kolbens zu verhindern. Hierdurch kann ein Verstopfen des Ventils und/oder der Durchführungen verhindert werden. Die poröse Schicht kann beispielsweise ein Metalldrahtgeflecht oder ein Netz aus einem Kunststoffgewebe aufweisen. Die poröse Schicht kann auch eine mit zweckmäßig dimensionierten Löchern versehene Scheibe aus Metall oder Kunststoff aufweisen. Weiterhin kann die poröse Schicht offenporige Schaumstoffe wie zum Beispiel Porex aufweisen. Bevorzugt kann die poröse Schicht aus einem Material gefertigt sein, welches durch die in der Vorrichtung verwendete Monomerflüssigkeit nicht angegriffen wird.

In einer Ausführungsform ist die Vorrichtung ausgebildet und eingerichtet, durch Ausübung einer Kraft auf den ersten Raum und/oder den zweiten Raum Knochenzement aus der Vorrichtung nach außen abzugeben. Hierzu kann die Kartusche einen beweglichen ersten Kolben und einen beweglichen zweiten Kolben aufweisen. Durch Ausübung von Druck auf den ersten Kolben kann dieser zunächst in Richtung des zweiten Kolbens bewegt werden, sodass der zweite Kolben von dem ersten Kolben mitgenommen wird, und dadurch einen Druckaufbau im zweiten Raum bewirkt. Hierdurch kann der Knochenzement aus dem zweiten Raum der Kartusche gepresst werden.

In einer Ausführungsform weist die Vorrichtung weiterhin ein Auspresselement vor. Das Auspresselement ist bevorzugt zum Verschieben des ersten Kolbens und/oder des zweiten Kolbens ausgebildet und eingerichtet. Das Auspresselement kann eine Stange aufweisen, welche zur Übertragung einer Kraft auf den ersten Kolben ausgebildet und eingerichtet ist. In einer Ausführungsform weist das Auspresselement eine Gewindestange auf. Die Kartusche kann ein Verbindungsmittel zur mechanischen Verbindung mit dem Auspresselement aufweisen. Das Verbindungsmittel kann beispielsweise ein Gewinde aufweisen. In einer Ausführungsform ist das Verbindungsmittel ausgebildet und eingerichtet, eine drehfeste Verbindung mit dem Auspresselement zu bilden. Das Auspresselement ist bevorzugt ausgebildet und eingerichtet, Knochenzement aus dem zweiten Raum der Kartusche zu pressen.

Das Auspresselement kann eine Gewindestange mit einem Außengewinde aufweisen. In einer Ausführungsform beträgt eine Steigung des Außengewindes wenigstens 1 mm, bevorzugt wenigstens 3 mm und/oder höchstens 7 mm, bevorzugt höchstens 5 mm. Dies hat sich in Versuchen als besonders zielführend erwiesen. In einer Ausführungsform beträgt ein Außendurchmesser des Außengewindes der Gewindestange wenigstens 12 mm und/oder höchstens 17 mm. Der Außendurchmesser bedeutet den maximalen Durchmesser, der zwischen den zwei maximalen Erhebungen des Gewindegangs gemessen wird, also den Durchmesser eines das Außengewinde umgebenden, gedachten Kreiszylinders. Insbesondere beträgt der Außendurchmesser wenigstens 13,5 mm und/oder höchstens 15 mm. In einer Ausführungsform beträgt der Außendurchmesser etwa oder genau 14 mm. Ein Außendurchmesser unterhalb von 12 mm ist nicht geeignet, den auftretenden Kräften bzw. Momenten in geeigneter Weise standzuhalten. Ein zu großer Außendurchmesser erhöht die Reibung und damit die für ein Austragen benötigte Kraft. In einer Ausführungsform enthält das Innengewinde der Adaptereinheit wenigstens 2 Gewindegänge, bevorzugt wenigstens oder genau 4 Gewindegänge und/oder höchstens 6 Gewindegänge, bevorzugt höchstens 5 Gewindegänge. In einer Ausführungsform ist das Gewinde der Gewindestange ein Trapezgewinde. Dies hat sich als besonders geeignet erwiesen. In einer Ausführungsform weist das Außengewinde der Gewindestange eine Gewindetiefe von wenigstens 1 mm und/oder höchstens 3 mm oder 4 mm auf.

Die Kartusche kann weiterhin eine Aufnahme zur Verbindung mit einem Auspresselement aufweisen Das Auspresselement kann eine Tellerscheibe aufweisen, die ausgebildet und eingerichtet ist, in eine Aufnahme der Kartusche einzugreifen. Die Kartusche kann weiterhin ein Kartuschenkopf aufweisen. Die oben beschriebene Aufnahme kann an diesem Kartuschenkopf angeordnet sein. Wie oben dargestellt, kann das Auspresselement ein Gewinde aufweisen. Der Kartuschenkopf kann ein Gewinde aufweisen, dass ausgebildet und eingerichtet ist, in ein Gewinde des Auspresselements einzugreifen. In einer Ausführungsform ist die Vorrichtung ausgebildet und eingerichtet, mithilfe des Auspresselement den ersten Kolben in die Kartusche zu schieben, um eine Monomerflüssigkeit aus dem ersten Raum der Kartusche durch das Ventil in den zweiten Raum der Kartusche abzugeben.

In einer Ausführungsform weist der Vorratsbehälter weiterhin ein Öffnungselement auf, das zum Brechen einer Glasampulle oder zum Öffnen eines Folienbeutels innerhalb des Vorratsbehälters ausgebildet und eingerichtet ist.

In einer Ausführungsform weist der Vorratsbehälter weiterhin ein Bedienelement auf, um das Öffnungselement zu steuern. Bevorzugt ist das Bedienelement operativ mit dem Öffnungselement verbunden. Der Vorratsbehälter kann weiterhin eine Sicherung aufweisen, welche das Bedienelement vor einer unbeabsichtigten Betätigung schützt. Eine solche Sicherung kann beispielsweise eine Klemme oder ein Rastelement aufweisen.

Der Vorratsbehälter kann ein erstes Ende und ein zweites Ende aufweisen. Das Bedienelement kann an dem ersten Ende des Vorratsbehälters angeordnet sein.

In einer Ausführungsform ist das Bedienelement in Richtung des zweiten Endes des Vorratsbehälters beweglich. Hierdurch kann ein Gefäß mit einer Monomerflüssigkeit, beispielsweise eine Glasampulle oder ein Folienbeutel, in Kontakt mit einem Öffnungselement gebracht werden, um das Gefäß zu öffnen. Das Öffnungselement kann beispielsweise einen Vorsprung, einen Dorn oder ein Messer aufweisen. In einer Ausführungsform weist der Vorratsbehälter ein Öffnungselement auf, welches einen Vorsprung aufweist, der ausgebildet und eingerichtet ist, den Hals einer Glasampulle durch Druck gegen den Vorsprung aufzubrechen, um die Glasampulle zu öffnen.

Der Vorratsbehälter kann einen Träger beweisen, der zur Aufnahme eines versiegelten Gefäßes mit einer Monomerflüssigkeit ausgebildet und eingerichtet ist. In einer Ausführungsform ist das Bedienelement ausgebildet und eingerichtet, den Träger in Richtung eines Öffnungselements zu bewegen.

In einer Ausführungsform ist der Vorratsbehälter mehrteilig ausgebildet. Der Vorratsbehälter kann ein flexibles Teilelement und ein starres Teilelement aufweisen. Der Vorratsbehälter kann ausgebildet und eingerichtet sein, ein Gefäß mit einer Monomerflüssigkeit zu öffnen, indem eine Kraft auf das flexible Teilelement ausgeübt wird, sodass das flexible Teilelement sich relativ zu dem starren Teilelement bewegt oder sich das flexible Teilelement verformt. Ein Beispiel dieses Funktionsprinzips ist ausführlich in der EP2404864B1 beschrieben.

Ein weiterer Aspekt betrifft die Verwendung einer hierin beschriebenen Vorrichtung zur Herstellung eines Knochenzements. Die Vorrichtung kann wie hierin beschrieben verwendet werden, um aus einer Monomerflüssigkeit und einem Knochenzementpulver Knochenzement herzustellen. Hierbei kann bevorzugt die Monomerflüssigkeit innerhalb der Vorrichtung freigesetzt und mit dem Knochenzementpulver zu einem Knochenzement gemischt werden. Die Vorrichtung kann weiterhin einen Schnorchel zum Ausbringen von Knochenzement aufweisen. Der Schnorchel ist bevorzugt mit der Kartusche verbindbar. In einer Ausführungsform ist der Schnorchel lösbar mit der Kartusche verbindbar. Die Kartusche kann ein Gewinde zur Verbindung mit einem solchen Schnorchel aufweisen. Die Kartusche kann einen Auslass aufweisen, der fluidleitend mit dem zweiten Raum der Kartusche verbunden ist. Der Schnorchel kann bevorzugt mit einem solchen Auslass verbindbar sein.

Die Kartusche kann weiterhin einen Verschluss aufweisen. Der Verschluss kann mit einem Gewinde versehen sein. Der Verschluss kann lösbar mit der Kartusche verbindbar sein. In einer Ausführungsform ist der Verschluss am zweiten Ende der Kartusche angeordnet. In einer Ausführungsform ist der Verschluss mit einem oben beschriebenen Auslass verbindbar, bevorzugt lösbar verbindbar.

In einer Ausführungsform wird ein Kit bereitgestellt, welche einen hierin beschriebenen Vorratsbehälter und eine hierin beschriebene Kartusche aufweist, wobei das Kit weiterhin einen Verschluss und einen Schnorchel aufweist, wobei der Verschluss und der Schnorchel jeweils zueinander austauschbar mit einem Auslass der Kartusche verbindbar sind.

Ein weiterer Aspekt betrifft ein Verfahren zur Herstellung eines Knochenzements, aufweisend die nachfolgenden Schritte,
(a) Bereitstellen einer hierin beschriebenen Vorrichtung,
(b) Freisetzen einer Monomerflüssigkeit aus einem versiegelten Gefäß innerhalb des Vorratsbehälters und Abgabe der Monomerflüssigkeit an den ersten Raum der Kartusche,
(c) Halten der Monomerflüssigkeit innerhalb des ersten Raums der Kartusche, wobei ein Kontakt der Monomerflüssigkeit mit dem zweiten Raum der Kartusche vermieden wird,
(d) Druckbeaufschlagen der Monomerflüssigkeit in dem ersten Raum und dadurch Öffnen des Ventils und Abgeben der Monomerflüssigkeit aus dem ersten Raum der Kartusche durch das Ventil in den zweiten Raum der Kartusche.

Die in diesem Verfahren verwendete Vorrichtung weist bevorzugt einen Vorratsbehälter zur Aufnahme eines versiegelten Gefäßes mit einer Monomerflüssigkeit, sowie weiterhin eine Kartusche mit einem ersten Raum und einen zweiten Raum auf, wobei der zweite Raum zur Aufnahme eines Knochenzementpulvers ausgebildet und eingerichtet ist, und wobei der erste Raum über ein Ventil mit dem zweiten Raum fluidleitend verbunden ist.

In einer Ausführungsform erfolgt das Halten der Monomerflüssigkeit innerhalb des ersten Raums der Kartusche für einen Zeitraum von länger als 1 Minute und weniger als 1 Stunde.

Das Verfahren kann weiterhin das Mischen einer Monomerflüssigkeit, eines Knochenzementpulvers und eines Antibiotikums umfassen. Beispiele für geeignete Antibiotika umfassen Gentamycin, Vancomycin und Daptomycin.

Das Verfahren kann weiterhin die Herstellung eines Knochenzements durch Vermischen der Monomerflüssigkeit mit einem Knochenzementpulver umfassen. Dieser Mischvorgang findet bevorzugt innerhalb des zweiten Raums der Kartusche statt. Das Verfahren kann weiterhin die Abgabe eines Knochenzements aus der Vorrichtung umfassen. Die Abgabe des Knochenzements kann *in vitro* erfolgen, oder die Abgabe kann unmittelbar an einen Patienten erfolgen, wobei der Knochenzement unmittelbar mit Patientengewebe in Kontakt gebracht wird. Das Verfahren kann weiterhin die Herstellung eines Spacers aus einem in der erfindungsgemäßen Vorrichtung hergestellten Knochenzement umfassen. Hierzu kann der Knochenzement zunächst in der Vorrichtung hergestellt und danach an eine Gießform für einen Spacer abgegeben werden.

Ein weiterer Aspekt der Erfindung betrifft ein Behandlungsverfahren, wobei ein mithilfe einer hierin beschriebenen Vorrichtung hergestellter Knochenzement an einen Patienten abgegeben wird. Das Behandlungsverfahren kann die Implantation eines künstlichen Gelenks oder eines Spacers umfassen.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### Figuren

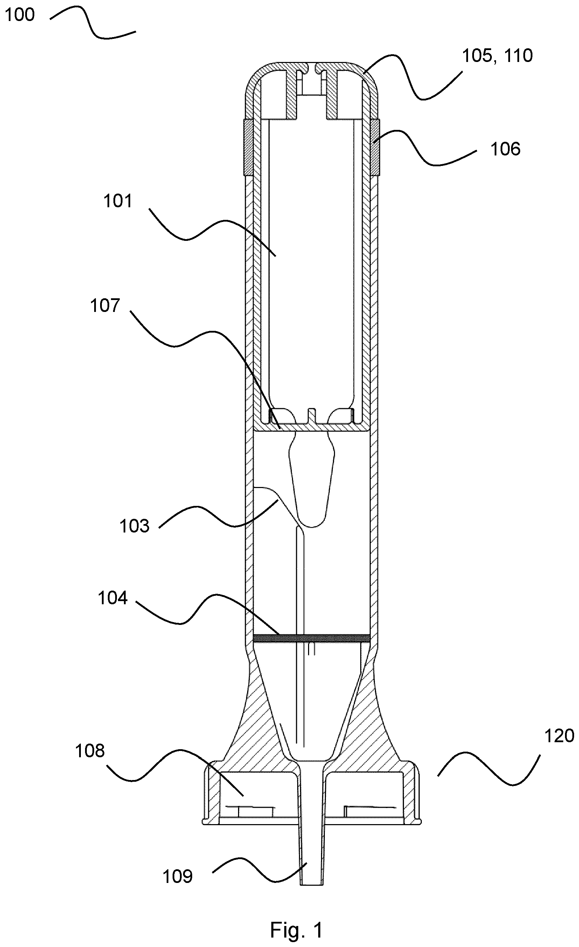

**Figur 1** zeigt einen Vorratsbehälter **100** einer erfindungsgemäßen Vorrichtung. Der Vorratsbehälter **100** weist hier ein Gehäuse mit einer im Wesentlichen zylindrischen Form auf. Der Vorratsbehälter **100** weist ein erstes Ende **110** und ein gegenüberliegendes zweites Ende **120** auf. Der Vorratsbehälter **100** weist weiterhin einen Träger **107** auf. Der Träger **107** ist zum Halten eines Gefäßes **101** mit einer Monomerflüssigkeit **102** ausgebildet und eingerichtet. Im hier gezeigten Beispiel ist das Gefäß **101** als Glasampulle ausgestaltet. Die Glasampulle ist in dem Träger **107** angeordnet. Der Hals der Glasampulle ragt in der hier gezeigten Anordnung aus dem Träger **107** hervor. Der Träger **107** ist beweglich in dem Vorratsbehälter **100** angeordnet, sodass eine Bewegung des Gefäßes **101** ermöglicht wird, um dieses zu öffnen. Diese Bewegung erfolgt über ein Bedienelement **105,** welches operativ mit dem Träger **107** verbunden ist. Das Bedienelement **105** ist an dem ersten Ende **110** des Vorratsbehälters angeordnet. Durch Bewegung des Bedienelements **105** ist der Träger **107,** und somit das darin angeordnete Gefäß **101,** in Richtung des zweiten Endes des Vorratsbehälters beweglich. Hierdurch kann das Gefäß **101** in Kontakt mit einem Öffnungselement **103** gebracht werden, um das Gefäß zu öffnen. Das Öffnungselement **103** ist hier als Vorsprung ausgestaltet, der innerhalb des Vorratsbehälters **100** angeordnet ist. Durch Bewegung des Bedienelements **105** kann somit das Gefäß **101** geöffnet werden. Im hier gezeigten Beispiel kann der Hals der Glasampulle **101** durch Druck gegen den Vorsprung **103** von der Ampulle **101** abgebrochen werden, um die Ampulle zu öffnen. Der Vorratsbehälter **100** weist weiterhin einen Filter **104** auf, um Bruchstücke eines geöffneten Gefäßes zurückzuhalten. Hierbei kann es sich beispielsweise um Glasbruchstücke einer geöffneten Glasampulle oder um Teile eines geöffneten Folienbeutels handeln. Der Vorratsbehälter **100** weist weiterhin an seinem zweiten Ende **120** ein erstes Verbindungselement **108** auf, das zur flüssigkeitsdichten Verbindung des Vorratsbehälters **100** mit einer Kartusche **200** ausgebildet und eingerichtet ist. Der Vorratsbehälter **100** weist weiterhin an seinem zweiten Ende **120** einen Stutzen **109** auf, der zur Abgabe einer Flüssigkeit an eine Kartusche **200** ausgebildet und eingerichtet ist. Der Vorratsbehälter **100** weist hier einen Innenraum auf, der sich am zweiten Ende **120** in Richtung des Stutzens **109** verjüngt. Die hier gezeigte Ausführungsform des Vorratsbehälters **100** weist weiterhin eine Sicherung **106** auf, welche das Bedienelement **105** vor einer unbeabsichtigten Betätigung und Bewegung schützt. Die Sicherung **106** ist hier als Sicherungsklemme ausgestaltet, welche den Vorratsbehälter **100** teilweise umgibt, und die Bewegung des Bedienelements **105** begrenzt. Die Sicherung **106** ist lösbar mit dem Vorratsbehälter **100** verbunden. Nach Entfernen der Sicherung **106** kann das Bedienelement **105** bewegt werden, um das Gefäß **101** mithilfe des Öffnungselements **103** zu öffnen.

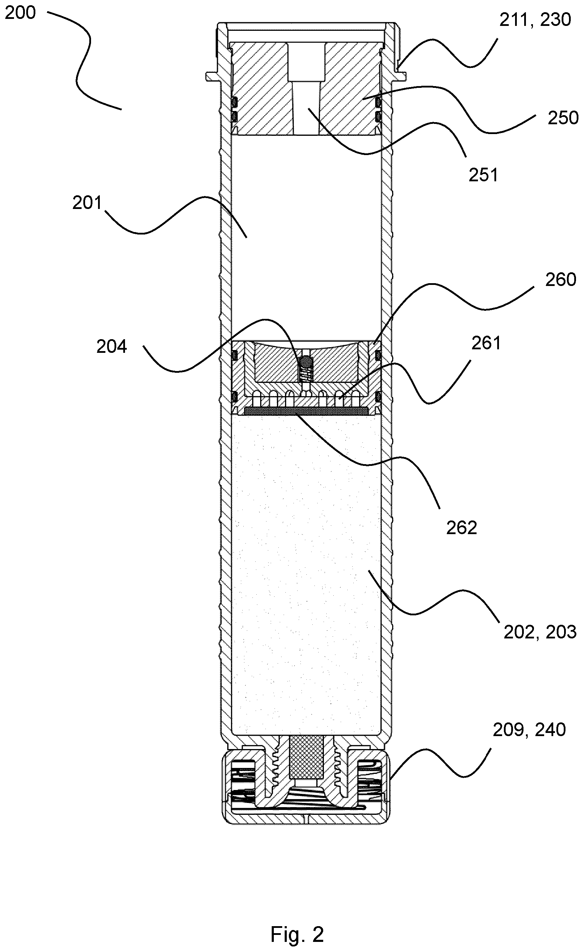

**Figur 2** zeigt eine Kartusche **200** einer erfindungsgemäßen Vorrichtung. Die Kartusche **200** weist ein erstes Ende **230** und ein zweites Ende **240** auf. Die Kartusche **200** weist einen ersten Raum **201** und einen zweiten Raum **202** auf. In der hier gezeigten Ausführungsform ist der erste Raum **201** am ersten Ende **230** angeordnet, und der zweite Raum **202** ist am zweiten Ende **240** angeordnet. Der erste Raum **201** ist zur Aufnahme einer Monomerflüssigkeit aus einem erfindungsgemäßen Vorratsbehälter ausgebildet und eingerichtet. Am ersten Ende **230** ist ein erster Kolben **250** angeordnet. Der erste Kolben **250** ist beweglich in der Kartusche **200** angeordnet, und begrenzt den ersten Raum **201** der Kartusche nach außen. Der erste Kolben **250** weist eine erste Durchführung **251** auf. Die erste Durchführung **251** ist ausgebildet und eingerichtet, eine formschlüssige fluidleitende Verbindung mit dem Stutzen **109** auszubilden. Der zweite Raum **202** ist zur Aufnahme eines Knochenzementpulvers **203** ausgebildet und eingerichtet. Im hier gezeigten Beispiel ist ein Knochenzementpulver **203** in dem zweiten Raum **202** angeordnet. Zwischen dem ersten Raum **201** und im zweiten Raum **202** der Kartusche ist ein zweiter Kolben **260** beweglich in der Kartusche **200** angeordnet. Der zweite Kolben **260** weist ein Ventil **204** auf, das eine fluidleitende Verbindung zwischen dem ersten Raum **201** und dem zweiten Raum **202** der Kartusche bildet. Das Ventil **204** ist unidirektional. Dies bedeutet, dass eine Monomerflüssigkeit aus dem ersten Raum **201** durch das Ventil **204** in den zweiten Raum **202** fließen kann, aber keine Monomerflüssigkeit oder Knochenzementpulver aus dem zweiten Raum **202** in den ersten Raum **201** gelangen kann. Das Ventil **204** ist druckabhängig, sodass eine Monomerflüssigkeit in einem drucklosen Zustand in dem ersten Raum **201** gehalten werden kann, und in einem druckbeaufschlagten Stand aus dem ersten Raum **201** in den zweiten Raum **202** abgegeben werden kann.

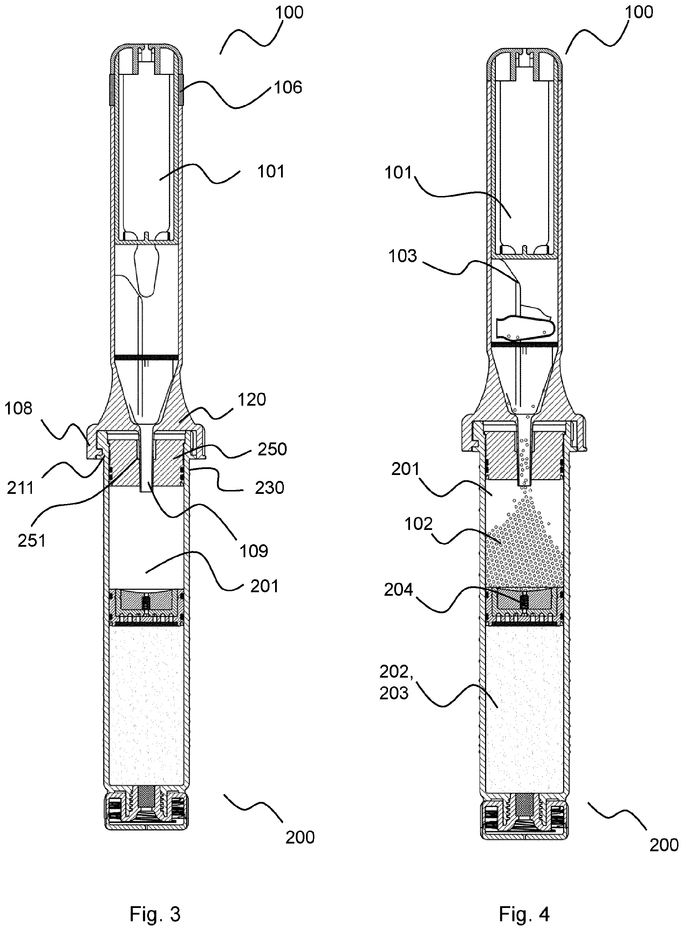

**Figur 3** zeigt eine erfindungsgemäße Vorrichtung, wobei ein Vorratsbehälter **100** mit einer Kartusche **200** in Kontakt gebracht ist. Das zweite Ende **120** des Vorratsbehälters ist dabei unmittelbar mit dem ersten Ende **230** der Kartusche verbunden. Hierzu weist der Vorratsbehälter **101** erstes Verbindungselement **108** auf, welches in ein zweites Verbindungselement **211** der Kartusche **200** eingreift. In der hier gezeigten Ausführungsform bilden das erste Verbindungselement **108** und das zweite Verbindungselement **211** gemeinsam eine formschlüssige Verbindung nach Art eines Bajonettverschluss aus. Der Stutzen **109** ist in eine erste Durchführung **251** des ersten Kolbens eingeführt, um eine fluidleitende Verbindung zwischen dem Vorratsbehälter **100** und dem ersten Raum **201** der Kartusche herzustellen. Eine Sicherung **106** verhindert die Bewegung des Bedienelements **105.** Auf diese Weise kann eine unbeabsichtigte Öffnung des Gefäßes **101** verhindert werden.

**Figur 4** zeigt eine erfindungsgemäße Vorrichtung, wobei eine Monomerflüssigkeit **102** aus dem Vorratsbehälter **100** in einen ersten Raum **201** der Kartusche **200** überführt ist. Mithilfe eines Öffnungselements **103** ist das Gefäß **101** geöffnet worden, um die Monomerflüssigkeit **102** zunächst innerhalb des Vorratsbehälters **100** freizusetzen und anschließend an den ersten Raum **201** der Kartusche abzugeben. Die Abgabe der Monomerflüssigkeit **102** an den ersten Raum **201** kann ohne Druckbeaufschlagung erfolgen, beispielsweise durch freien Gravitationsfluss. Im hier gezeigten Zustand der Vorrichtung ist die Monomerflüssigkeit **102** im ersten Raum der Kartusche **201** gehalten. Die Monomerflüssigkeit **102** weist hierbei keine Druckbeaufschlagung auf. Hierbei wird ein Kontakt der Monomerflüssigkeit mit dem zweiten Raum **202** der Kartusche **200** vermieden, da das Ventil **204** im drucklosen Zustand der Monomerflüssigkeit **102** geschlossen ist. Dies erlaubt eine gesteuerte Abgabe der Monomerflüssigkeit aus dem ersten Raum **201** an den zweiten Raum **202** der Kartusche und eine Lagerung der Monomerflüssigkeit innerhalb des ersten Raums **201.** Die Monomerflüssigkeit **102** kann dabei über einen Zeitraum von beispielsweise einigen Minuten in dem ersten Raum **201** gehalten werden, ohne dass eine chemische Reaktion der Monomerflüssigkeit **102** mit Komponenten der Außenluft oder Knochenzementpulver erfolgt.

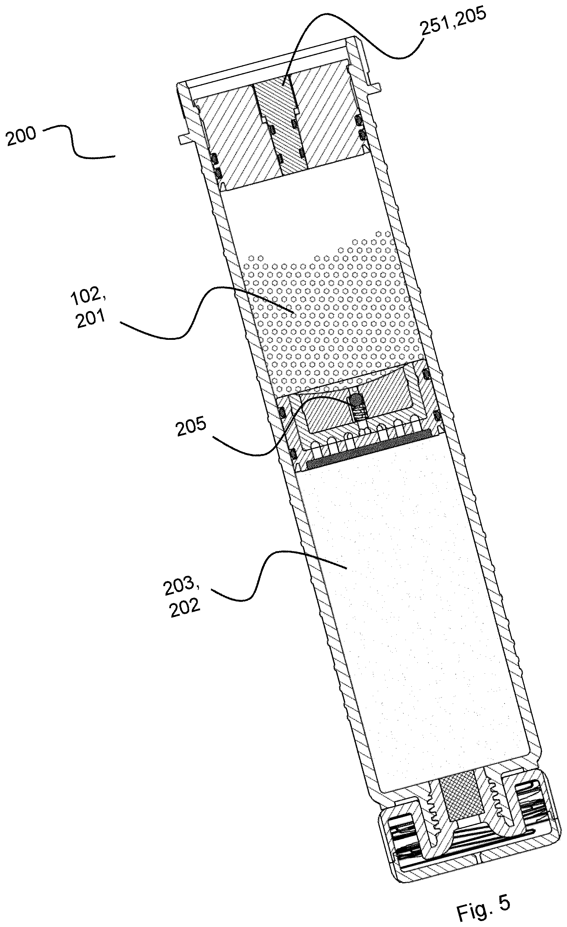

**Figur 5** zeigt eine Kartusche **200** mit einem ersten Kolben **250,** der durch einen Stopfen **205** verschlossen ist. Der hier gezeigte Zustand der Kartusche **200** kann erreicht werden, nachdem, wie beispielsweise in Figur 3 gezeigt, ein Gefäß mit einer Monomerflüssigkeit in dem Vorratsbehälter geöffnet und, wie beispielsweise in Figur 4 gezeigt, an den ersten Raum **201** abgegeben wurde. Eine Monomerflüssigkeit **102** wird im ersten Raum **201** gehalten. Das Ventil **205** ist geschlossen, da sich die Monomerflüssigkeit in einem nicht druckbeaufschlagten Zustand befindet. Die erste Durchführung **251,** welche in dem ersten Kolben **250** angeordnet ist, ist durch einen Stopfen **205** verschlossen, um ein Austreten der Monomerflüssigkeit **102** aus dem ersten Raum **201** zu verhindern. Somit kann die Monomerflüssigkeit **102** in dem ersten Raum **201** gehalten werden. Dies ermöglicht dem Benutzer der Vorrichtung, die Monomerflüssigkeit zwischenzulagern und für die Vermischung mit einem Knochenzementpulver **203,** das im zweiten Raum **202** angeordnet ist, bis zu einem gewünschten Zeitpunkt bereitzuhalten. Im hier gezeigten Beispiel weist der Stopfen **205** umlaufende Gummidichtungen auf, um den ersten Raum **201** flüssigkeitsdicht nach außen zu begrenzen.

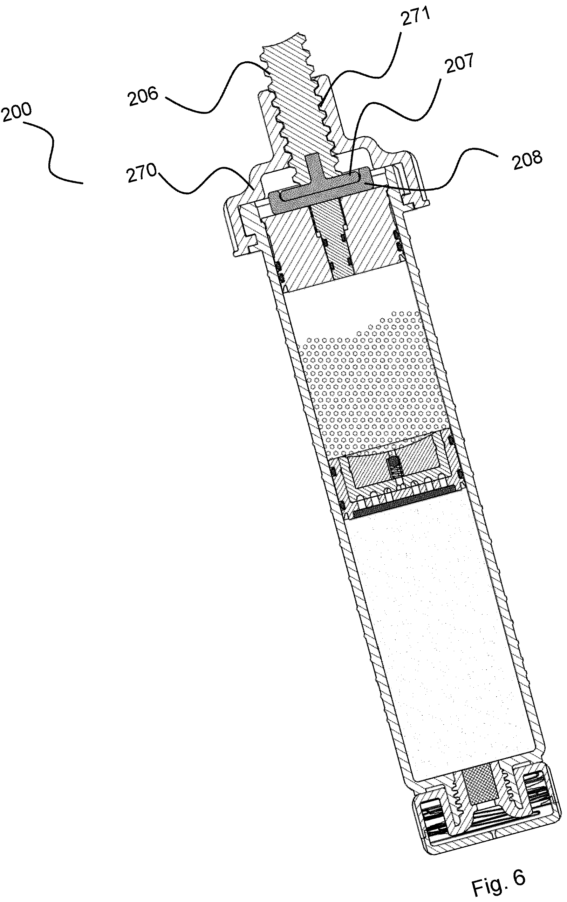

**Figur 6** zeigt eine Kartusche **200,** die im Eingriff mit einer Auspressstange **206** steht. Die Auspressstange **206** weist eine Tellerscheibe **207** auf, welche in eine Aufnahme **208** eingreift, die am ersten Kolben **250** angeordnet ist. Am ersten Ende der Kartusche **230** weist die Kartusche **200** einen Kartuschenkopf **270** auf. Der Kartuschenkopf **270** weist ein innenliegendes Gewinde **271** auf. Die Auspressstange **206** kann in das Gewinde **271** eingreifen, um eine Kraft auf den ersten Kolben **250** auszuüben. Somit kann mithilfe der Auspressstange **206** der erste Kolben **250** in die Kartusche **200** geschoben werden, um eine Monomerflüssigkeit **102** aus dem ersten Raum der Kartusche **201** durch das Ventil **204** in den zweiten Raum **202** der Kartusche abzugeben.

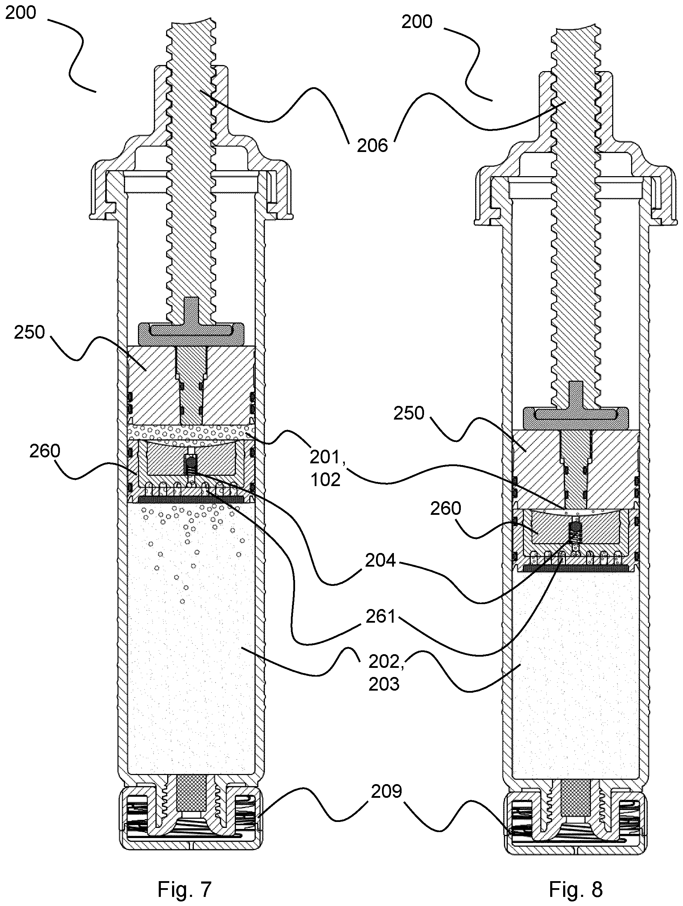

**Figur 7** zeigt eine Kartusche **200,** bei welcher ein erster Kolben **250** mithilfe einer Auspressstange **206** in Richtung eines zweiten Kolbens **260** bewegt ist. Die Bewegung des ersten Kolbens **250** führt zu einer Verkleinerung des Innenvolumens des ersten Raums **201,** sodass die enthaltene Monomerflüssigkeit **102** druckbeaufschlagt wird. Hierdurch öffnet sich das druckabhängige Ventil **204,** sodass die Monomerflüssigkeit **102** aus dem ersten Raum **201** an den zweiten Raum **202** der Kartusche abgegeben wird. Die Monomerflüssigkeit **102** wird hierbei durch das Ventil **204,** eine zweite Durchführung **261** und eine poröse Schicht **262** geleitet. Die poröse Schicht dient als Filter, da sie für Luft und die Monomerflüssigkeit durchlässig ist, aber für das Knochenzementpulver undurchlässig ist. Innerhalb des zweiten Raums **202** gelangt die Monomerflüssigkeit **102** hierdurch in Kontakt mit einem Knochenzementpulver **203,** wobei aus der Monomerflüssigkeit und dem Knochenzementpulver ein Knochenzement gebildet wird.

**Figur 8** zeigt eine Kartusche, bei welcher ein erster Kolben **250** mithilfe einer Auspressstange **206** gegen einen zweiten Kolben **260** gedrückt ist. Hierdurch verkleinert sich das Innenvolumen des ersten Raums **201** der Kartusche auf ein minimales Volumen, das auch als Totvolumen bezeichnet wird. Die Monomerflüssigkeit **102** ist im Wesentlichen vollständig aus dem ersten Raum **201** an den zweiten Raum **202** der Kartusche abgegeben worden. Durch die weitere Bewegung des ersten Kolbens **250** wird auch der zweite Kolben **260** in Richtung des zweiten Endes der Kartusche bewegt. Hierdurch erfolgt eine Druckbeaufschlagung des Inhalts des zweiten Raums **202** der Kartusche. Der zweite Raum **202** ist durch einen Verschluss **209** nach außen begrenzt. Der Verschluss **209** ist vorliegend als Schraubverschluss ausgebildet. Zur Verbindung mit diesem Schraubverschluss weist die Kartusche am zweiten Ende **240** ein Gewinde auf.

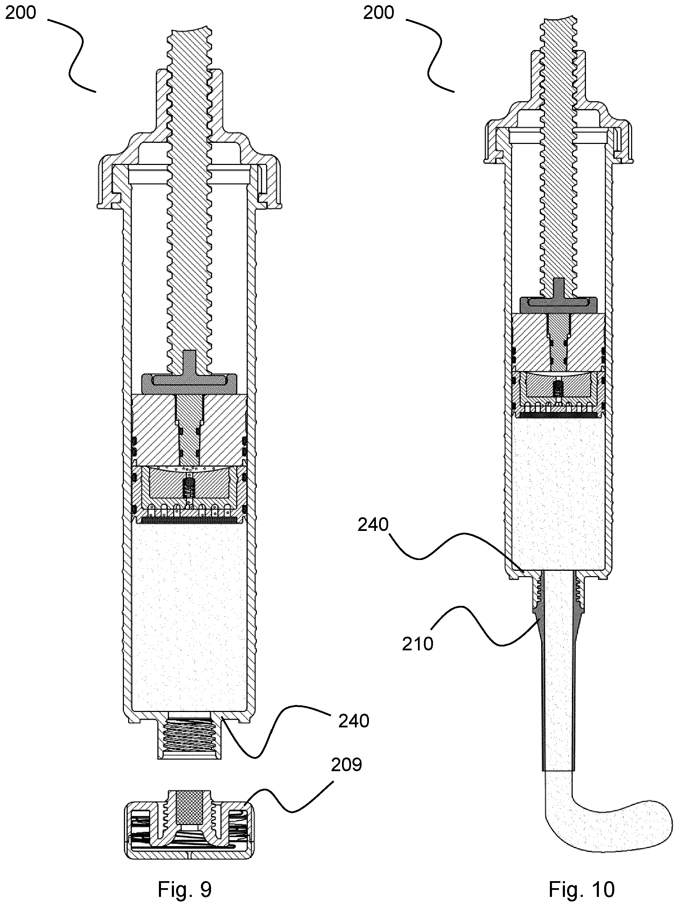

**Figur 9** zeigt eine Kartusche **200** mit fertig gemischtem Knochenzement, wobei die Kartusche durch Entfernen eines Verschlusses **209** geöffnet ist. Dies ermöglicht eine Abgabe des Knochenzements aus der Kartusche.

**Figur 10** zeigt eine Kartusche mit fertig gemischtem Knochenzement, wobei die Kartusche einen Schnorchel **210** zum Ausbringen von Knochenzement aufweist. Vorliegend weist die Kartusche am zweiten Ende **240** ein Gewinde auf, welches das Aufschrauben des Schnorchels **210** an die Kartusche ermöglicht.

### Bezugszeichenliste

- **100**: Vorratsbehälter
- **101**: Gefäß
- **102**: Monomerflüssigkeit
- **103**: Öffnungselement
- **104**: Filter
- **105**: Bedienelement
- **106**: Sicherung
- **107**: Träger
- **108**: erstes Verbindungselement
- **109**: Stutzen
- **110**: erstes Ende des Vorratsbehälters
- **120**: zweites Ende des Vorratsbehälters
- **200**: Kartusche
- **201**: erster Raum der Kartusche
- **202**: zweiter Raum der Kartusche
- **203**: Knochenzementpulver
- **204**: Ventil
- **205**: Stopfen
- **206**: Auspressstange
- **207**: Tellerscheibe
- **208**: Aufnahme für Tellerscheibe 207
- **209**: Verschluss
- **210**: Schnorchel
- **211**: zweites Verbindungselement
- **230**: erstes Ende der Kartusche
- **240**: zweites Ende der Kartusche
- **250**: erster Kolben
- **251**: erste Durchführung
- **260**: zweiter Kolben
- **261**: zweite Durchführung
- **262**: poröse Schicht
- **270**: Kartuschenkopf
- **271**: Gewinde des Kartuschenkopfs

## Patentansprüche

1. Vorrichtung zur Herstellung eines Knochenzements, aufweisend einen Vorratsbehälter (100) zur Aufnahme eines versiegelten Gefäßes (101) mit einer Monomerflüssigkeit (102), und eine Kartusche (200) zum Mischen von Knochenzement, wobei die Kartusche (200) einen ersten Raum (201) zur Aufnahme von Monomerflüssigkeit aus dem Vorratsbehälter (100) und einen zweiten Raum (202) zur Aufnahme eines Knochenzementpulvers (203) aufweist, und wobei die Kartusche weiterhin ein Ventil (204) aufweist, welches den ersten Raum (201) mit dem zweiten Raum (202) fluidleitend verbindet,
wobei die Vorrichtung ausgebildet und eingerichtet ist,
in einem ersten Schritt innerhalb des Vorratsbehälters (100) eine Monomerflüssigkeit aus einem versiegelten Gefäß (101) freizusetzen und an den ersten Raum (201) der Kartusche (200) abzugeben,
in einem zweiten Schritt die Monomerflüssigkeit innerhalb des ersten Raums (201) zu halten, wobei ein Kontakt der Monomerflüssigkeit mit dem zweiten Raum (202) der Kartusche (200) vermieden wird,
und in einem dritten Schritt die Monomerflüssigkeit aus dem ersten Raum (201) der Kartusche (200) in den zweiten Raum (202) der Kartusche (200) abzugeben.

2. Vorrichtung nach Anspruch 1, wobei der Vorratsbehälter (100) und die Kartusche (200) als jeweils voneinander getrennte Elemente ausgestaltet sind, welche lösbar miteinander verbindbar sind.

3. Vorrichtung nach einem der vorangehenden Ansprüche, wobei die Kartusche (200) weiterhin einen verschiebbaren ersten Kolben (250) aufweist, der mit dem Vorratsbehälter (100) verbindbar ist.

4. Vorrichtung nach Anspruch 4, wobei der erste Kolben (250) eine erste Durchführung (251) zur Abgabe einer Monomerflüssigkeit aus dem Vorratsbehälter (100) den ersten Raum (201) der Kartusche (202) aufweist.

5. Vorrichtung nach einem der vorangehenden Ansprüche, wobei die Kartusche (200) weiterhin einen verschiebbaren zweiten Kolben (260) aufweist, welcher zwischen dem ersten Raum (201) und dem zweiten Raum (202) der Kartusche (200) angeordnet ist.

6. Vorrichtung nach Anspruch 5, wobei das Ventil (204) in dem zweiten Kolben (260) angeordnet ist.

7. Vorrichtung nach Anspruch 6, wobei der zweite Kolben (260) eine Vielzahl von Durchführungen (261) aufweist, welche eine fluidleitende Verbindung zwischen dem Ventil (204) und dem zweiten Raum (202) der Kartusche (200) ausbilden.

8. Vorrichtung nach einem der Ansprüche 5 bis 7, wobei der zweite Kolben weiterhin eine fluidleitende poröse Schicht (262) aufweist, welche eingerichtet ist, das Eindringen von Knochenzementpulver aus dem zweiten Raum (202) der Kartusche (200) in das Ventil (204) und/oder ggf. die Durchführungen (261) nach Anspruch 7 zu verhindern.

9. Vorrichtung nach einem der vorangehenden Ansprüche, wobei das Ventil (204) ausgebildet und eingerichtet ist, in einem drucklosen Zustand des ersten Raums (201) eine Monomerflüssigkeit vollständig in dem ersten Raum (201) zu halten, und in einem druckbeaufschlagten Zustand des ersten Raums (201) eine Monomerflüssigkeit an den zweiten Raum (202) abzugeben.

10. Vorrichtung nach einem der vorangehenden Ansprüche, wobei das Ventil (204) bevorzugt ein durch Druckbeaufschlagung zu öffnendes Ventil ist, wobei das Ventil weiter bevorzugt eine Feder und einen an der Feder gelagerten Dichtkörper aufweist, oder einen verklemmten, durch Druckbeaufschlagung lösbaren Dichtkörper aufweist, oder wobei das Ventil ein Bunsenventil ist.

11. Vorrichtung nach einem der vorangehenden Ansprüche, welche ausgebildet und eingerichtet ist, durch Ausübung einer Kraft auf den ersten Raum (201) und/oder den zweiten Raum (202) Knochenzement aus der Vorrichtung nach außen abzugeben.

12. Vorrichtung nach einem der vorangehenden Ansprüche, weiterhin aufweisend ein Auspresselement, wobei das Auspresselement ggf. zum Verschieben des ersten Kolben (261) und/oder des zweiten Kolben (261) ausgebildet und eingerichtet ist.

13. Vorrichtung nach einem der vorangehenden Ansprüche, wobei der Vorratsbehälter (100) weiterhin ein Öffnungselement (103) aufweist, das zum Brechen einer Glasampulle oder zum Öffnen eines Folienbeutels innerhalb des Vorratsbehälters (100) ausgebildet und eingerichtet ist.

14. Vorrichtung nach einem der vorangehenden Ansprüche, wobei die Vorrichtung ausgebildet und eingerichtet ist, eine Monomerflüssigkeit durch freien Gravitationsfluss aus dem Vorratsbehälter (100) an den ersten Raum (201) der Kartusche (200) abzugeben.

15. Verwendung einer Vorrichtung nach einem der vorangehenden Ansprüche zur Herstellung eines Knochenzements.

16. Verfahren zur Herstellung eines Knochenzements, aufweisend die nachfolgenden Schritte (a) bis (d),
(a) Bereitstellen einer Vorrichtung gemäß einem der Ansprüche 1 bis 14,
(b) Freisetzen einer Monomerflüssigkeit aus einem versiegelten Gefäß (101) innerhalb des Vorratsbehälters (100) und Abgabe der Monomerflüssigkeit an den ersten Raum (201) der Kartusche (200),
(c) Halten der Monomerflüssigkeit innerhalb des ersten Raums (201) der Kartusche (200), wobei ein Kontakt der Monomerflüssigkeit mit dem zweiten Raum (202) der Kartusche (200) vermieden wird,
(d) Druckbeaufschlagen der Monomerflüssigkeit in dem ersten Raum (201) und dadurch Öffnen des Ventils (204) und Abgeben der Monomerflüssigkeit aus dem ersten Raum (201) der Kartusche (200) durch das Ventil (204) in den zweiten Raum (202) der Kartusche (200).
